# EUROPEAN PATENT APPLICATION

(11) **EP 4 732 844 A1**
(43) Date of publication of application: **29.04.2026**
(21) Application number: 24831935.2
(22) Date of filing: 25.06.2024
(51) Int. Cl.: A61K 36/48, A23L 33/105, A61P 27/16

(54) **COMPOSITION FOR TREATING AND/OR PREVENTING HEARING IMPAIRMENT**

(30) Priority: 26.06.2023 JP 2023104149
(71) Applicant: Otsuka Pharmaceutical Co., Ltd., Tokyo 101-8535 (JP)
(72) Inventor: KOBAYASHI, Yodai, Osaka-shi, Osaka 540-0021 (JP); KOUDA, Noriyuki, Osaka-shi, Osaka 540-0021 (JP); INOUE, Syoichiro, Osaka-shi, Osaka 540-0021 (JP); KAWAHARA, Tomohiro, Osaka-shi, Osaka 540-0021 (JP); KOTANI, Yoshifumi, Osaka-shi, Osaka 540-0021 (JP); HAMURO, Koji, Osaka-shi, Osaka 540-0021 (JP)
(74) Representative: Hoffmann Eitle
(86) International application number: PCT/JP2024/022948
(87) International publication number: WO 2025/005075

(57) **Abstract**

The present disclosure provides a composition for treating and/or preventing hearing impairment comprising an asparagus extract, or a composition for maintaining and/or improving hearing function, comprising an asparagus extract.

## Description

### TECHNICAL FIELD

This patent application claims the priority of Japanese Patent Application No. 2023-104149, the entire disclosure of which is incorporated herein by reference.

The present disclosure relates to a composition for treating and/or preventing hearing impairment, or to a composition for maintaining and/or improving hearing function.

### BACKGROUND ART

The World Health Organization (WHO) has reported that more than one billion young people are at risk of noise-induced hearing loss due to exposure to loud sound for purposes such as listening to music. Age-related hearing loss is considered to be a major risk factor for dementia, and the prevention of hearing loss is also important as the prevention of dementia. Noise-induced and age-related hearing loss are thought to result from gradual damage to hair cells in inner ear caused by noise or aging. Hearing loss is difficult to be cured because hair cells are hardly regenerated, and preventive care in daily life is required. Tinnitus is a sensory abnormality in which sound is perceived in the absence of a sound stimulus, and the psychological burden caused by tinnitus and related symptoms is considerable. Hearing loss is thought to be one of the factors that induce tinnitus.

Asparagus is a vegetable produced and consumed in many countries, and has been reported to have various physiological activities. For example, it has been found that extracts from asparagus stems exhibit preventive and restorative effects against various types of fatigue (e.g., physical fatigue and stress-induced fatigue) and improve brain function, whereas extracts from asparagus pseudoleaves exhibit regulatory effects on the autonomic nervous system. Patent Document 1 describes that hydroxy-methyl-furfural derivatives obtained by heat-treating an asparagus with hot water have excellent HSP-inducing activity, anti-stress effects, and regulatory effects on the autonomic nervous system. However, no effect of asparagus extract on hearing impairment or hearing function has been reported.

### PRIOR ART DOCUMENT

### PATENT DOCUMENT

Patent Document 1: WO 2013/094676

### SUMMARY OF THE INVENTION

### PROBLEMS TO BE SOLVED BY THE INVENTION

The purpose of the present disclosure is to provide a method for treating and/or preventing hearing impairment, or a method for maintaining and/or improving hearing function.

### MEANS FOR SOLVING THE PROBLEMS

The inventors have found that an asparagus extract is effective in improving hearing loss and tinnitus.

Accordingly, in an aspect, the present disclosure provides a composition for treating and/or preventing hearing impairment comprising an asparagus extract.

In another aspect, the present disclosure provides a composition for maintaining and/or improving hearing function comprising an asparagus extract.

### EFFECTS OF THE INVENTION

The present disclosure provides novel means for treating and/or preventing hearing impairment, or for maintaining and/or improving hearing function.

### BRIEF DESCRIPTION OF THE DRAWINGS

[Fig. 1] Fig. 1 shows the results of an ABR test in a mouse model of age-related hearing loss that was fed either a control diet or a test diet containing an asparagus extract and exposed to a sound stimulus of 32 kHz.
[Fig.2] Fig.2 shows the total THI scores in a clinical study examining the effects of asparagus extract intake on tinnitus in healthy subjects, comparing the asparagus extract group and the placebo group.

### DETAILED DESCRIPTION

Unless otherwise defined, the terms used herein are read as generally understood by those skilled in the fields such as organic chemistry, medical sciences, pharmaceutical sciences, molecular biology, and microbiology. Definitions of several terms used herein are provided below, and these definitions shall prevail over the general understanding herein.

When a numerical value is preceded by the term "about", the value is intended to represent any value in the range of -10% of the value to +10% of the value. For example, "about 20" means "a value from 18 to 22." A numerical range includes all values and the endpoints between the two limits. The term "about," when used in connection with a range, applies to both endpoints of that range. For example, "about 20 to 30" includes "18 to 33."

The asparagus extract of the present disclosure is a mixture of asparagus-derived components contained in a liquid obtained by subjecting asparagus to hot-water extraction and removing the residue. The raw material for the asparagus extract is preferably the stem portion of Asparagus officinalis (asparagus stem), including young stems and leaves. The asparagus stems may be lower portions of edible asparagus that are discarded for reasons of harvesting, transportation, or sales efficiency. The raw material for the asparagus extract may be fresh asparagus, dried asparagus, or powdered dried asparagus. Examples of asparagus include green asparagus, white asparagus, and purple asparagus, preferably green asparagus. The place of production of asparagus is not particularly limited.

The method for producing the asparagus extract of the present disclosure includes a step of subjecting asparagus to hot-water extraction. The term "hot-water extraction" means heating the raw material in hot water to extract components contained in the raw material into water. The step of subjecting asparagus to hot-water extraction can be carried out, for example, by adding 1 to 50 times the amount of water to asparagus and heating it in hot water for 20 to 180 minutes. The temperature in this step is preferably 50 to 300 °C. When performing hot-water extraction under atmospheric pressure, for example, a temperature of 90 °C or higher is preferred. Hot-water extraction may also be performed under pressure; for example, extraction at 0.1 to 0.2 MPa (e.g., 0.12 MPa when using an autoclave) is preferred.

The method for producing the asparagus extract of the present disclosure may further include, for the purpose of improving the efficiency of the step of hot-water extraction, one or more additional steps as exemplified below.

As an example of the additional step, the asparagus may be cut into smaller pieces before the hot-water extraction. The asparagus can be cut, for example, into pieces having a size of about 0.1 to 10 cm. The cutting may be performed manually using a knife or cutter, or mechanically using a cutting machine or mill.

As an example of the additional step, the asparagus may be pressed before the hot-water extraction. The pressing can be performed, for example, using a press machine.

As an example of the additional step, an enzymatic treatment may be performed before or after the step of hot-water extraction for the purpose of, for example, destroying plant tissues. The enzymatic treatment can further improve the efficiency of the step of hot-water extraction. For example, to efficiently decompose fibrous materials or pectin in asparagus, enzymes such as cellulase, hemicellulase, pectinase, amylase, or pullulanase, or combinations of two or more of these enzymes, may be suitably used. In the step of enzymatic treatment, the optimum amount of enzyme, temperature, and reaction time may be selected depending on the enzyme used. When cellulase is used, for example, enzymatic treatment can be performed at a temperature of 30 to 60 °C for 1 to 72 hours by adding cellulase in an amount of 0.1 to 5% (w/w). When pectinase is used, for example, enzymatic treatment can be performed at a temperature of 30 to 60 °C for 1 to 72 hours by adding pectinase in an amount of 0.1 to 10% (w/w). The step of enzymatic treatment may either before or after the step of hot-water extraction. It is preferable to perform enzymatic treatment with cellulase and/or pectinase after the step of hot-water extraction.

As an example of the additional step, the residue may be mechanically ground after the hot-water extraction. The grinding may be performed mechanically using a mill, mixer or the like.

As an example of the additional step, centrifugation or filtration may be carried out after the hot-water extraction. Such an additional step may efficiently remove the residue to provide a heat-treated liquid. Centrifugation may be carried out, for example, at 3,000 to 7,000 rpm and at 4 to 50 °C. For filtration, for example, commercially available filter paper, filter cloth or the like may be used.

As an example of the additional step, the heat-treated liquid obtained after the hot-water extraction may be concentrated under reduced pressure. The concentration can be carried out using, for example, an evaporator or the like.

As an example of the additional step, the heat-treated liquid obtained after the hot-water extraction may be subjected to spray drying or freeze drying. Spray drying may be carried out, for example, at an exhaust air temperature of 70 to 90 °C and a chamber temperature of 80 to 100°C. A filler may be added to the heat-treated liquid prior to spray drying or freeze drying. Examples of fillers include starch, starch hydrolysates, dextrin, maltodextrin, maltooligosaccharide, lactose, sorbitol, mannitol, and sucrose. After spray drying or freeze drying, the resulting product may be sieved.

In an embodiment, the method for producing the asparagus extract includes the following steps:
(1) subjecting asparagus to hot-water extraction to obtain a hot-water extract;
(2) treating the hot-water extract with an enzyme to obtain an enzymatically treated product;
(3) removing the residue from the enzymatically treated product to obtain an enzymatically treated liquid; and
(4) concentrating, spray drying, and/or freeze drying the enzymatically treated liquid to obtain the asparagus extract.

In an embodiment, the method for producing the asparagus extract includes the following steps:
(1) treating asparagus with an enzyme to obtain an enzymatically treated product;
(2) subjecting the enzymatically treated product to hot-water extraction to obtain a hot-water extract;
(3) removing the residue from the hot-water extract to obtain a heat-treated liquid; and
(4) concentrating, spray drying, and/or freeze drying the heat-treated liquid to obtain the asparagus extract.

Examples of the method for producing the asparagus extract are as follows: Production Example 1: Asparagus stems are cut into pieces about 0.1 to 10 cm in length, and 1 to 50 times the amount of water is added. The cut asparagus stems are subjected to hot-water extraction at 50 to 100 °C or under pressure at 121 °C for 20 to 180 minutes. After cooling, cellulase is added in an amount of 0.1 to 5% (w/w), and the extract is treated with the enzyme at 30 to 60 °C for 1 to 72 hours. The residue is mechanically ground, and the product is centrifuged at 3,000 to 7,000 rpm and 4 to 50 °C to obtain the supernatant. The supernatant is spray-dried at an exhaust air temperature of 70 to 90 °C and a chamber temperature of 80 to 100 °C.

Production Example 2: Asparagus stems are cut into pieces about 0.1 to 10 cm in length, and 1 to 50 times the amount of water is added. The cut asparagus stems are subjected to hot-water extraction at 50 to 100 °C or under pressure at 121 °C for 20 to 180 minutes. After cooling, cellulase is added in an amount of 0.1 to 5% (w/w), and the extract is treated with the enzyme at 30 to 60 °C for 1 to 72 hours. Pectinase is added in an amount of 0.1 to 10% (w/w) and the extract is treated with the enzyme at 30 to 60 °C for 1 to 72 hours. The enzyme is inactivated by heat. The product is centrifuged at 3,000 to 7,000 rpm and 4 to 50 °C to obtain the supernatant. The supernatant is concentrated and a filler is added. The concentrate is sterilized under pressure and then spray-dried at an exhaust air temperature of 70 to 90 °C and a chamber temperature of 80 to 100 °C, or freeze-dried.

Production Example 3: Dried asparagus stems are pulverized into powder, and 1 to 50 times the amount of water is added. The powdered asparagus stems are subjected to hot-water extraction at 50 to 100 °C or under pressure at 121 °C for 20 to 180 minutes. After cooling, cellulase and pectinase are added in an amount of 0.1 to 5% w/w and 0.1 to 10% w/w, respectively, and the extract is treated with the enzymes at 30 to 60 °C for 1 to 72 hours. The enzymes are inactivated by heat. The product is centrifuged at 3,000 to 7,000 rpm and 4 to 50 °C to obtain the supernatant. The supernatant is concentrated and a filler is added. The concentrate is sterilized under pressure and then spray-dried at an exhaust air temperature of 70 to 90 °C and a chamber temperature of 80 to 100 °C, or freeze-dried.

Typically, the amount of aspartic acid contained in the asparagus extract is 10% or less, 5% or less, 4% or less, 3% or less, 2% or less, 1% or less, 0.5% or less, 0.3%, or 0.25% or less.

As shown in the Examples below, the composition of the present disclosure can treat and/or prevent hearing impairment. In the present disclosure, hearing impairment includes hearing loss and tinnitus. In an embodiment, the hearing impairment is not associated with another disease such as Meniere's disease.

Hearing loss is one of the most common sensory disorders and is characterized by a decline in hearing ability. Hearing loss includes age-related hearing loss, noise-induced hearing loss, and drug-induced hearing loss, particularly age-related hearing loss. Examples of noise-induced hearing loss include acoustic hearing loss and occupational hearing loss. Hearing loss can be diagnosed by tests such as pure-tone audiometry, speech audiometry, word recognition score, tympanometry, and stapedial reflex.

Tinnitus is the perception of sound in the absence of a sound stimulus. Tinnitus may be evaluated, for example, by the Tinnitus Handicap Inventory (THI). The THI consists of 25 items, and for each item, the subject chooses one of the following responses: "often (4 points)," "sometimes (2 points)," or "never (0 points)." The total score is used to determine the severity of tinnitus-related distress. The original version of the THI is in English, and reliable, validated translations, including a Japanese version, are available. A higher THI score indicates greater tinnitus distress; scores of 18 to 36 are classified as mild, 38 to 56 as moderate, and 58 to 100 as severe. In an embodiment, the tinnitus is classified as mild or moderate by the THI.

As used herein, "treating" or "treatment" refers to delaying or stopping the progression of hearing impairment and/or reducing, alleviating, improving, or removing the hearing impairment in a subject having hearing impairment.

In the present disclosure, "preventing" or "prevention" refers to preventing the onset of hearing impairment or reducing the likelihood of developing hearing impairment in a subject who is at risk of developing hearing impairment but has not yet developed it. Subjects who are at risk of developing hearing impairment but have not yet developed it include subjects having risk factors for hearing impairment. Examples of the risk factors for hearing impairment include advanced age (e.g., 50 years or older, 55 years or older, 60 years or older, 65 years or older, 70 years or older, or 75 years or older), exposure to noise or loud sounds, changes in air pressure, stress, accumulation of earwax, impaired blood flow in the inner ear, viral infection, drugs (e.g., antibiotics, anticancer drugs, salicylate drugs), acoustic neuroma, otitis media (e.g., chronic otitis media, otitis media with effusion), head trauma, autoimmune diseases, and genetics.

The subject for treating and/or preventing hearing impairment may be an animal, generally a mammal such as a human, a non-human primate (e.g., monkey, gibbon, chimpanzee, orangutan, or macaque), a companion animal (e.g., dog or cat), livestock (e.g., horse, cattle, goat, sheep, or pig), or a laboratory animal (e.g., mouse, rat, rabbit, or guinea pig), preferably a human. The age of the human subject is not limited, but may be, for example, middle-aged or older, specifically 50 years or older, 55 years or older, 60 years or older, 65 years or older, 70 years or older, or 75 years or older. In an embodiment, the subject is not afflicted with Meniere's disease.

As shown in the Examples below, the composition of the present disclosure can maintain and/or improve hearing function. In the present disclosure, maintenance of hearing function includes maintaining hearing ability and suppressing its decline. Improvement of hearing function includes improving hearing ability, maintaining the improved hearing ability, and suppressing decline from the improved hearing ability. Hearing ability can be measured by pure-tone audiometry.

The subject for maintaining and/or improving hearing function may be an animal, generally a mammal such as a human, a non-human primate (e.g., monkey, gibbon, chimpanzee, orangutan, or macaque), a companion animal (e.g., dog or cat), livestock (e.g., horse, cattle, goat, sheep, or pig), or a laboratory animal (e.g., mouse, rat, rabbit, or guinea pig), preferably a human. The age of the human subject is not limited, but may be, for example, middle-aged or older, specifically 50 years or older, 55 years or older, 60 years or older, 65 years or older, 70 years or older, or 75 years or older. The subject may or may not have hearing impairment. In an embodiment, the subject is not afflicted with Meniere's disease.

The composition of the present disclosure may be a pharmaceutical composition or a quasi-drug composition. The asparagus extract may be formulated by conventional methods into dosage forms such as tablets, granules, powders, capsules, syrups, or injections. Additives commonly used in pharmaceuticals, such as fillers, binders, lubricants, colorants, disintegrants, thickeners, preservatives, stabilizers, and pH adjusters, may be added. The route of administration of the pharmaceutical or quasi-drug composition is not particularly limited and may be oral, intravenous, intraperitoneal, intradermal, or sublingual, preferably oral.

The composition of the present disclosure may also be a food composition. The food composition may be in a general processed food form, for example, a solid food or a liquid food such as a beverage, drink, powdered drink, or soup. The food composition may be consumed as a food product such as juice, confectionery, jelly, tablets, dressing, or seasoning. The food composition may be produced by formulating the asparagus extract into a form suitable for ingestion, such as granules, particles, tablets, capsules, gels, creams, pastes, suspensions, aqueous solutions, emulsions, or powders, by conventional methods. Additives commonly used in foods, such as fillers, binders, lubricants, colorants, disintegrants, thickeners, preservatives, stabilizers, and pH adjusters, may be added. Furthermore, a component such as sugars, sugar alcohols, salts, fats and oils, amino acids, organic acids, or glycerin, may be added for improving taste. The asparagus extract may also be added to existing foods, and the base food is not particularly limited.

The food may also be provided as a food with health claims or a dietary supplement. Examples of foods with health claims include foods for specified health uses, foods with nutrient function claims, and foods with functional claims. Foods with health claims may be labeled as being intended for use in maintaining and/or improving hearing function. The labeling may be made directly on the package, container, label, tag, or accompanying documents associated with the product, or indirectly through advertising activities. Examples of dietary supplements include nutritional supplements and health supplements.

The dosage and method of administration of the composition, or the ingestion amount and method of ingestion of the composition, are not particularly limited. They may be determined in consideration of factors such as the subject's sex, age, body weight, general health condition, and pathological condition. For example, the dosage or ingestion amount of the composition of the present disclosure may be about 50 mg to 2000 mg, about 50 mg to 1350 mg, about 100 mg to 1000 mg, about 100 mg to 500 mg, about 100 mg to 300 mg, about 100 mg to 200 mg, about 110 mg to 190 mg, about 120 mg to 180 mg, about 130 mg to 170 mg, about 140 mg to 160 mg, or about 145 mg to 155 mg of asparagus extract per day, for example about 150 mg. The daily dosage or ingestion amount of asparagus extract may be contained in a single composition, or may be distributed across multiple compositions administered or ingested daily.

The composition of the present disclosure may be administered or ingested once or multiple times. In the case of multiple administrations or ingestions, the composition may be administered or ingested once or several times per day, for example once, twice, or three times per day, either every day or every few days, such as every 1, 2, 3, or 7 days. The period of administration or ingestion is not limited and the composition may be continuously administered or ingested, for example, at least one week, preferably at least four weeks, and more preferably at least eight weeks. There may be periods during which administration or ingestion is discontinued. In an embodiment, the composition of the present disclosure is administered or ingested for at least eight weeks. In an embodiment, the composition of the present disclosure is administered or ingested daily.

The composition of the present disclosure may be used alone or in combination with one or more additional components. "Combination" refers not only to the use of a dosage form containing all components or the combined use of dosage forms containing the components separately, but also to simultaneous administration or ingestion of the components, or delayed administration or ingestion of one or more of the components, so long as they are used for the same purpose. Two or more additional components may also be used in combination. For example, a composition containing one or more additional components in addition to the asparagus extract may be used. Examples of components suitable for combination include diuretics, vasodilators, vitamins, steroids, anti-vertigo agents, ginkgo biloba extract, bee larvae extract, and Kampo medicines.

In addition to administration or ingestion of the asparagus extract, non-pharmacological medical interventions may also be performed, such as tinnitus retraining therapy, the use of hearing aids, or cochlear implantation.

An aspect provides a method of treating and/or preventing hearing impairment comprising administering an effective amount of an asparagus extract to a subject in need thereof.

An aspect of the disclosure provides an asparagus extract for use in treating and/or preventing hearing impairment.

An aspect of the disclosure provides use of an asparagus extract for treating and/or preventing hearing impairment.

An aspect of the disclosure provides use of an asparagus extract for manufacturing a composition for treating and/or preventing hearing impairment.

An aspect provides a method of maintaining and/or improving hearing function comprising administering an effective amount of an asparagus extract to a subject in need thereof.

An aspect of the disclosure provides an asparagus extract for use in maintaining and/or improving hearing function.

An aspect of the disclosure provides use of an asparagus extract for maintaining and/or improving hearing function.

An aspect of the disclosure provides use of an asparagus extract for manufacturing a composition for maintaining and/or improving hearing function.

For example, the disclosure provides the following embodiments.
[1] A composition for treating and/or preventing hearing impairment, comprising an asparagus extract.
[2] The composition according to item 1, wherein the hearing impairment is hearing loss or tinnitus.
[3] The composition according to item 1, wherein the hearing impairment is age-related hearing loss, noise-induced hearing loss, or tinnitus.
[4] The composition according to any one of items 1 to 3, wherein the hearing impairment is age-related hearing loss.
[5] The composition according to any one of items 1 to 3, wherein the hearing impairment is tinnitus.
[6] The composition according to item 5, wherein the tinnitus is classified as mild or moderate by the Tinnitus Handicap Inventory (THI).
[7] A composition for maintaining and/or improving hearing function, comprising an asparagus extract.
[8] The composition according to any one of items 1 to 7, which is a pharmaceutical composition, a quasi-drug composition, or a food composition.
[9] The composition according to any one of items 1 to 8, wherein 50 mg to 2000 mg of the asparagus extract is ingested per day.
[10] The composition according to any one of items 1 to 9, wherein the composition is ingested daily for at least eight weeks.
[11] The composition according to any one of items 1 to 10, wherein the asparagus extract is manufactured by a process comprising subjecting asparagus to hot-water extraction.
[12] The composition according to item 11, wherein the process further comprises treating the product of the hot-water extraction with an enzyme.
[13] The composition according to any one of items 1 to 12, wherein the asparagus extract is an asparagus stem extract.

### EXAMPLES

### Production of Asparagus Extract

An asparagus extract produced by the following method was used: Green asparagus stems (fresh weight: 1100 kg) were cut and mixed with 1430 L of water, and subjected to pressurized sterilization (121 °C, 45 minutes) for the purpose of hot-water extraction. After cooling to 55 °C, 16.5 kg of cellulase (Sucrase C, Mitsubishi Chemical Corporation) was added, and the materials were treated with the enzyme at 58 °C for 1 hour. After further cooling to 45 °C, 11 kg of pectinase (Macerozyme A, Yakult Pharmaceutical Industry Co., Ltd.) was added, and the materials were treated with the enzyme at 48 °C for 24 hours. The enzymes were subsequently inactivated by heat (100 °C, 20 minutes). The product was centrifuged, and the resulting liquid was concentrated using a concentrator, yielding 320 kg of concentrated liquid. After adding 50 kg of filler (Pinedex, Matsutani Chemical Industry Co., Ltd.), the concentrated liquid was subjected to pressurized sterilization (121 °C, 45 minutes). After spray drying, 125 kg of the asparagus extract was obtained.

### Evaluation of Hearing Improvement Using a Mouse Model of Age-Related Hearing Loss

Male C57BL/6J mice (SPF, 10 weeks old; Japan Charles River Co., Ltd.) were used (12 mice per cohort). A preliminary breeding period was set: 5 days for cohort 1, 6 days for cohort 2, and 7 days for cohort 3. During this period, body weight was measured three times, general appearance was observed once daily, and an ABR test was conducted once. Animals without abnormalities in weight progression, ABR (auditory brainstem response), or general appearance were included in the grouping.

In each cohort, the two animals with the highest total auditory threshold (sum of thresholds at 16, 24, and 32 kHz) at the end of the preliminary period were excluded (if three or more animals met the criteria, the two with the higher 32 kHz thresholds were excluded). The animals were divided into groups by randomization so that the mean and variance of body weight were approximately equal among the groups.

From the time of animal arrival to grouping, the animals were provided ad libitum access to feed (CRF-1, Oriental Yeast Co., Ltd.) in feeders. After grouping, the animals were fed either a control diet (Group 1: CRF-1) or a test diet (Group 2: CRF-1 containing 0.26% asparagus extract) ad libitum. Although asparagus is known to contain aspartic acid, which acts as a neurotransmitter, CRF-1 already contains 1.93 g/100 g of aspartic acid, and the asparagus extract of the present invention contains only a trace amount of aspartic acid; therefore, adding 0.26% asparagus extract to CRF-1 results in almost no change in aspartic acid content. The feed in the feeders was replaced with fresh feed at least once per week. The administration period was 12 weeks (85 days). General appearance was observed once daily in the morning.

### Auditory Brainstem Response (ABR) Test

ABR tests were conducted before administration (12 weeks of age) and at 8 and 12 weeks from the start of administration (20 and 24 weeks of age, respectively). A mixed-anesthetic solution [ketamine hydrochloride (75 mg/kg) and xylazine hydrochloride (10 mg/kg)] was administered subcutaneously into the back using a disposable polypropylene syringe (Terumo Corporation) equipped with a 25G needle (Terumo Corporation) (administration volume: 2 mL/kg). After anesthesia, the active electrode was placed near the external ear of the test ear (right ear), the reference electrode was placed subcutaneously on the vertex, and the ground electrode was placed subcutaneously on the dorsal neck. The ABR potential was induced from the electrodes into the bioelectric potential amplifier (ER-1, Cygnus Technology Inc.) and recorded with a system for data acquisition and analysis (PowerLab, Sampling soft: LabChart ver. 8, ADInstruments) (recording conditions: sampling time 10 ms, sampling rate 40 kHz, Bandpass filter 1-3000 Hz, 500 sweeps). A coupler-type speaker (ES1spc, Bio Research Center Co., Ltd.) was inserted into the right ear canal and sound stimuli were given by a TDT acoustic system (ZBus for system 3, Tucker-Davis Technologies Inc.) (sound pressure: 10 to 90 dB; tone-burst stimuli at 16, 24, and 32 kHz). First, a sound stimulus of 90 dB was given and the ABR was recorded. Sound pressure was adjusted in 5 dB steps to identify the maximum sound pressure at which the ABR waveform disappeared and the minimum sound pressure at which the ABR waveform was detected. The minimum sound pressure at which the ABR waveform was detected was defined as the sound pressure threshold (ABR thresholds; dB SPL). If no ABR waveform was detected at 90 dB, the sound pressure threshold was determined as 95 dB SPL.

### Statistical Analysis

For each group, the mean and standard error of sound pressure thresholds were calculated at each time point. For significance test for sound pressure threshold, Wilcoxon's signed-rank test was used to compare Pre vs. Week 6 and Pre vs. Week 12 in the control group to confirm age-related hearing loss was developed. Wilcoxon's rank-sum test was used to compare the control and test diet groups at each time point. A significance level of 5% was used. For significance test a commercial software program (SAS system, SAS Institute Japan Ltd.) was used (**: p < 0.01).

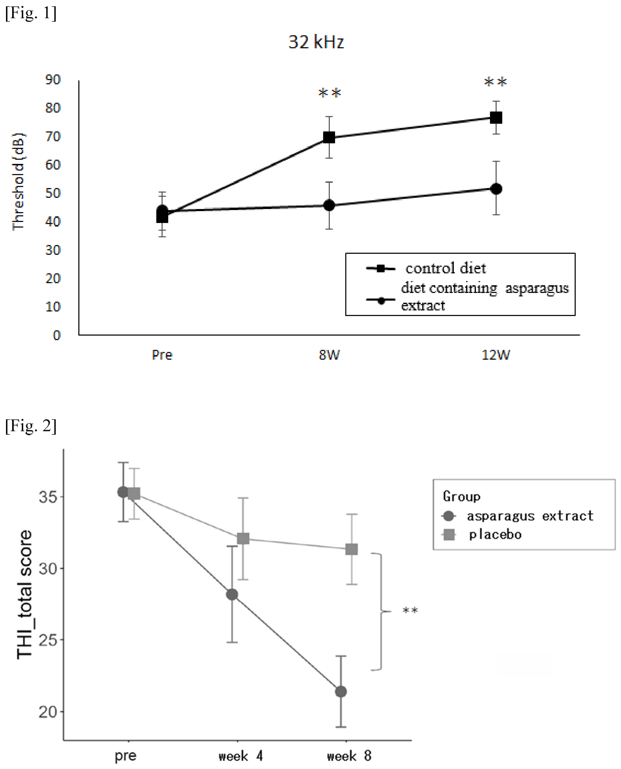

The results of the ABR test are shown in Fig. 1. When a sound stimulus was applied at 32 kHz, hearing ability declined with aging in the control group, whereas hearing ability was maintained in the test diet group. The results suggest that the asparagus extract is effective for the prevention and treatment of age-related hearing loss.

### Effect of Asparagus Extract Ingestion on Tinnitus in Healthy Subjects

A randomized, placebo-controlled, double-blind, parallel-group clinical trial was conducted to evaluate the effects of asparagus extract ingestion on tinnitus in healthy adult men and women.

The test enrolled healthy Japanese men and women aged 20 years or older who had subjective tinnitus and a score of 18 or higher and 56 or lower (mild to moderate tinnitus) on the Japanese version of the Tinnitus Handicap Inventory (Tinnitus Handicap Inventory; THI-J, Wakabayashi S, Oishi N, Shinden S, et al. Factor analysis and evaluation of each item of the tinnitus handicap inventory. Head Face Med. 2020;16(1):1-9).

The test-food group ingested 1.84 g of granulated food containing 150 mg of asparagus extract once daily for 8 weeks. The placebo group ingested 1.84 g of granulated food in which the asparagus extract had been replaced with cornstarch, caramel coloring, and dextrin once daily for 8 weeks. Both foods additionally contained sweeteners, acidulants, flavoring agents, and silicon dioxide.

THI-J assessments were conducted before ingestion, and at 4 and 8 weeks from the start of ingestion, and tinnitus severity was evaluated based on the total score. The results are shown in Fig. 2, which depicts the mean ± SEM of total scores in the test-food and placebo groups. Analysis was performed using a general linear mixed model (fixed effects: group, time, and group-by-time interaction; covariate: baseline score) (**: p < 0.01).

At week 8, the observed THI-J total score was significantly lower in the test-food group than in the placebo group. The THI is used internationally to evaluate tinnitus burden, and the validity and reliability of the Japanese version has been confirmed (Wakabayashi S, et al., 2020, supra). Higher scores indicate greater tinnitus distress; scores of 18 to 36 indicate mild, 38 to 56 moderate, and 58 to 100 severe tinnitus. A clinically meaningful improvement is known to be a change of at least 6 or 7 in total THI score (Japan Audiological Society, Clinical Practice Guidelines for the Diagnosis and Management of Tinnitus 2019, Kanehara & Co., Ltd., pp. 7-61; Zeman F, Koller M, Figueiredo R, et al. Tinnitus Handicap Inventory for Evaluating Treatment Effects. Otolaryngol Neck Surg. 2011;145(2):282-7). Difference in the total score of THI-J at week 8 was -10.9 points (95% CI [-17.7, -4.2]) between the groups. Changes from baseline were -14.8 ± 12.8 points in the test-food group and -3.9 ± 10.5 in the placebo group, with a difference between the groups of -10.9 points (95% CI [-17.7, -4.2]). Only the test food group showed a change of more than 7 points in the total THI-J score after the intervention, and the difference at week 8 between the groups was more than 7 points, indicating the significant difference between the groups is a clinically meaningful difference. The results suggest that the asparagus extract is effective for the prevention and treatment of tinnitus.

### INDUSTRIAL APPLICABILITY

The present disclosure relates to the treatment and/or prevention of hearing impairment or the maintenance and/or improvement of hearing function, and is applicable in the fields of medicine and food.

## Claims

1. A composition for treating and/or preventing hearing impairment, comprising an asparagus extract.

2. The composition according to claim 1, wherein the hearing impairment is hearing loss or tinnitus.

3. The composition according to claim 1, wherein the hearing impairment is age-related hearing loss, noise-induced hearing loss, or tinnitus.

4. The composition according to claim 1, wherein the hearing impairment is age-related hearing loss.

5. The composition according to claim 1, wherein the hearing impairment is tinnitus.

6. The composition according to claim 5, wherein the tinnitus is classified as mild or moderate by the Tinnitus Handicap Inventory (THI).

7. A composition for maintaining and/or improving hearing function, comprising an asparagus extract.

8. The composition according to any one of claims 1 to 7, which is a pharmaceutical composition, a quasi-drug composition, or a food composition.

9. The composition according to any one of claims 1 to 8, wherein 50 mg to 2000 mg of the asparagus extract is ingested per day.

10. The composition according to any one of claims 1 to 9, wherein the composition is ingested daily for at least eight weeks.

11. The composition according to any one of claims 1 to 10, wherein the asparagus extract is manufactured by a process comprising subjecting asparagus to hot-water extraction.

12. The composition according to claim 11, wherein the process further comprises treating the product of the hot-water extraction with an enzyme.

13. The composition according to any one of claims 1 to 12, wherein the asparagus extract is an asparagus stem extract.
